# EUROPEAN PATENT APPLICATION

(11) **EP 4 744 634 A1**
(43) Date of publication of application: **20.05.2026**
(21) Application number: 25213201.4
(22) Date of filing: 04.11.2025
(51) Int. Cl.: A61F 13/15

(54) **DEVICE AND METHOD FOR SEPARATING SEGMENTS FROM A CONTINUOUS WEB**

(30) Priority: 19.11.2024 IT 202400025980
(71) Applicant: GDM S.p.A., 40133 Bologna (IT)
(72) Inventor: ZAVALLONI, Alessandro, 40128 Bologna - Italy (IT)
(74) Representative: Puggioli, Tommaso

(57) **Abstract**

A separation method for segments (100) from a continuous web (200) comprises advancing the continuous web (200) in an advancement orientation (V) along a flat advancement path (P) at a first speed (V1), making at least one weakening line (101, 102, 103) on the continuous web (200), tearing the continuous web (200) at the first weakening line (101) by accelerating the continuous web (200).

## Description

The present invention relates to a segment separation device from a continuous web and a method, and in particular to a segment separation device from a continuous web and a method comprising absorbent material, in the manufacturing of diapers or the like.

Diapers and the like, such as underpads for beds or pets, comprise an absorbent pad that may comprise cellulose and superabsorbent material (SAP) dispersed therein.

Based on the desired final product, absorbent pads may be bagged in an external casing that may be, for example, made of fabric or of non-woven fabric.

The absorbent pads are formed by cutting a multilayer continuous web into segments, comprising all the layers of the pads and previously composed; downstream of the cut, the segments/pads are spaced at the pitch required by the format of the final product.

The main known solutions of methods and machines for separating the segments from the continuous web are distinguished into those in which the web is advanced in a plane by means of aspirated belts, is cut and the segments are spaced and advanced along the same plane and those in which the cutting of the web into segments and the spacing occur on an aspirated wheel.

A drawback of the known solutions of the first type derives from the fact that, especially for very thin pads, such as underpads, which are also wide, the newly cut segments tend to flutter due to the advancement speed and to lift from the belts, making it difficult to control the position and the advancement.

A drawback of the known solutions of the second type derives from the fact that the centrifugal force applied to the web and to the segments, due to the rotation of the aspirated wheel, may cause the dispersion of material, compromising the features of the product, in particular in the case of unbagged pads.

Therefore, the need is felt in the art for a segment separation device from a continuous web and method, which are more reliable than those known for any type of segment.

In this context, a segment separation method from a continuous web and device are suggested capable of overcoming at least some of the drawbacks of the aforesaid prior art and meeting the aforesaid need.

In particular, it is the object of the present invention to provide a segment separation device from a continuous web and method which allow good control of the positioning of the segments downstream of the cut, without dispersion of material from the continuous web and/or from the segments. This object is achieved by a segment separation device from a continuous web and method, comprising the technical features set out in one or more of the appended claims. The dependent claims correspond to possible different embodiments of the invention.

According to a first aspect, the present invention relates to a segment separation device from a continuous web.

The segment separation device from a continuous web comprises a flat advancement path for the continuous web.

The flat advancement path constitutes the path along which the continuous web advances and along which the segments are cut from the continuous web; preferably, the flat advancement path is horizontal.

The continuous web is, for example, a web comprising cellulose and superabsorbent material dispersed in the cellulose, and optionally a continuous casing of non-woven fabric or fabric.

The segments obtained from the web are preferably absorbent pads intended for absorbent articles, such as, for example, underpads or diapers, of the disposable type, for children or adults, of the type obtained by superposing a sheet of impermeable material (*backsheet*) onto a sheet of permeable material (*topsheet*) and interposing an absorbent pad between the sheets.

The separation device comprises a cutting knife arranged along the advancement path and configured to make weakening lines on the continuous web.

Each weakening line is transverse to the continuous web and located between consecutive segments according to an advancement orientation of the continuous web.

The separation device comprises, upstream of the cutting knife according to said advancement orientation, a feeding system for feeding the continuous web to the cutting knife at a first speed;

The feeding system comprises, for example, an aspirated belt, movable in the advancement orientation at the first speed, configured to withhold the continuous web and move it at the first speed.

The separation device comprises, downstream of the cutting knife according to the advancement orientation, a first aspirated belt, arranged along the advancement path.

The first aspirated belt is movable at a second speed greater than the first speed, and has an inlet and an outlet for the continuous web.

The first aspirated belt is configured to receive the continuous web, provided with weakening lines, and advance it along the advancement path.

Preferably, the first aspirated belt comprises a first aspirated part and a second aspirated part downstream of the first aspirated part and consecutive to the first aspirated part according to the advancement orientation.

The first aspirated part has a first negative pressure with a first intensity to withhold the continuous web on the first aspirated belt.

The second aspirated part has a second negative pressure with a second intensity to withhold the continuous web on the first aspirated belt.

The first and second intensities are, in practice, the aspiration intensity or aspiration force of the corresponding part of the aspirated belt, i.e., they represent the force with which the first and the second part of the aspirated belt withhold the continuous web.

The first intensity is lower than the second intensity so that the continuous web slides on the first aspirated part and is preferably movable at the first speed on the first aspirated part, and is preferably movable at the second speed with the second aspirated part towards the outlet of the first aspirated belt.

Advantageously, in this manner, the continuous web may be separated into segments, while being kept always adhered to support surfaces.

The continuous web may slide on the first aspirated part, remaining movable at the first speed, while the second aspirated part accelerates it, causing a tearing of the segment from the continuous web at a previously formed weakening line. The acceleration of the continuous web completes the cutting of the continuous web at the weakening line.

The continuous web slides on the first zone, and when the product advances sufficiently into the zone with greater aspiration, each segment separates from the one that follows in the advancement orientation.

The acceleration of the web and the separation of the segment also make it possible to space consecutive segments. Consecutive segments may be spaced at a desired pitch, for example and preferably, at the pitch at which they will further subsequently be applied to other webs.

The continuous web, even if it is thin or without a casing, always remains withheld and does not flutter; furthermore, not being subject to centrifugal force, it does not disperse material. By moving in the plane, any material that should lift will fall back onto the web itself.

According to an aspect, the first aspirated belt is immediately downstream of the cutting knife. In particular, the inlet of the first aspirated belt is immediately downstream of the cutting knife according to the advancement orientation.

Advantageously, the continuous web, provided with a first weakening line, is supported on the first belt immediately after the preparation of the weakening line. The second aspirated part may then tear off and space the segments obtaining the absorbent pads.

According to an aspect, the separation device may comprise a second aspirated belt, having a respective inlet and a respective outlet, arranged along the advancement path between the cutting knife and the first aspirated belt.

The second aspirated belt is preferably movable at said first speed and has a third negative pressure for withholding the continuous web to advance it along the advancement surface at the first speed.

Advantageously, the continuous web is not immediately divided into segments downstream of the cutting knife but is laid and conveyed by the second aspirated belt, once the weakening lines have been made, to the first aspirated belt.

Thereby, there is more time and space to handle the continuous web and the segments.

Preferably, a distance along the advancement path between the cutting knife and the inlet of the second aspirated belt is greater than a distance along the advancement path between the outlet of the second aspirated belt and the inlet of the first aspirated belt.

Given that the first aspirated belt may be positioned closer to the second aspirated belt than to the cutting knife, e.g., due to the dimension of the cutting knife, reducing the distance between the second aspirated belt and the first aspirated belt makes it possible to separate segments of reduced length.

The separation preferably occurs when the weakening line that is intended to be torn is on the first aspirated part of the first aspirated belt, and the weakened line consecutive to the weakening line which is intended to be torn according to the advancement orientation is still on the second aspirated belt.

According to an aspect, the present invention relates to a segment separation method from a continuous web.

The separation method comprises advancing the continuous web in an advancement orientation along a flat, preferably horizontal, advancement path at a first speed, and making at least one weakening line on the continuous web, transverse to the continuous web, and located between consecutive segments according to the advancement orientation.

The method comprises advancing the continuous web provided with the weakening lines at a first speed and tearing the continuous web at a first weakening line.

Preferably, the method comprises advancing on a first movable flat surface in the advancement orientation along the flat advancement path and advancing the continuous web on a second flat surface downstream of the first flat surface at a second speed greater than the first speed to tear the continuous web at a first weakening line. Advancing the continuous web on the second flat surface preferably occurs when the first weakening line is located on the first flat surface.

Preferably, the first flat surface and the second flat surface form a first aspirated belt movable at the second speed.

The separation method preferably comprises sliding and withholding the continuous web provided with weakening lines on the first movable flat surface, in the advancement orientation along the flat advancement path, and accelerating the continuous web on the second flat surface downstream of the first flat surface, to tear the continuous web at the first weakening line when the first weakening line is located on the first flat surface.

Preferably, tearing of the continuous web at a first weakening line occurs before making a second weakening line consecutive to the first weakening line according to the advancement orientation.

Preferably, the separation method comprises advancing the continuous web on a third flat surface at the first speed after making a sequence of weakening lines on the continuous web and before sliding the continuous web provided with the weakening lines on the first flat surface, the third flat surface being upstream of the first flat surface according to the advancement orientation.

Preferably, accelerating the continuous web on the second flat surface occurs when a consecutive second weakening line of the first weakening line according to the advancement orientation is located on the third flat surface.

Preferably, advancing the continuous web on the third flat surface comprises withholding the continuous web on the third flat surface and moving the third flat surface at the first speed.

Preferably, sliding the continuous web provided with the weakening lines on the first flat surface comprises withholding, with a first negative pressure, the continuous web on the first flat surface, and accelerating the continuous web on the second flat surface comprises withholding, with a second negative pressure, the continuous web on the third flat surface.

The first negative pressure is of lower intensity than the second negative pressure so that the continuous web slides on the first flat surface and is movable with the second flat surface at a second speed greater than the first speed. Advantageously, the separation method comprises moving the first and the second flat surface at the second speed greater than the first speed, and, by means of the first and the second negative pressure, imposing the first speed on the web on the first flat surface and the second speed on the second flat surface.

Preferably, the separation method is implemented with a device according to one of the preceding aspects.

According to an aspect, the present invention relates to a method for manufacturing an absorbent product comprising at least one permeable topsheet, an impermeable backsheet and an absorbent pad inserted between the topsheet and the backsheet.

The manufacturing method comprises a segment separation method from a continuous web according to a preceding aspect, to make a succession of absorbent pads spaced therebetween.

The manufacturing method comprises feeding the succession of absorbent pads spaced therebetween to an encounter with a second continuous web defining the backsheets and a third continuous web defining the topsheets to obtain a continuous composite web formed of the second continuous web, the first continuous web and the absorbent pads withheld between the first and second continuous webs.

Further features and advantages of the aforementioned aspects will be more apparent in the following indicative and consequently non-limiting description of a preferred, but not exclusive embodiment of a segment separation device from a continuous web and method.

This description will be presented below with reference to the appended drawings, provided for indicative purposes only and therefore non-limiting in which:
- figures 1 and 2 show a first embodiment of a segment separation device from a continuous web according to the description, in a diagrammatic side view and in two successive steps of a separation method according to the description;
- figures 3 and 4 show a second embodiment of a segment separation device from a continuous web according to the description, in a diagrammatic side view and in two successive steps of a separation method according to the description;

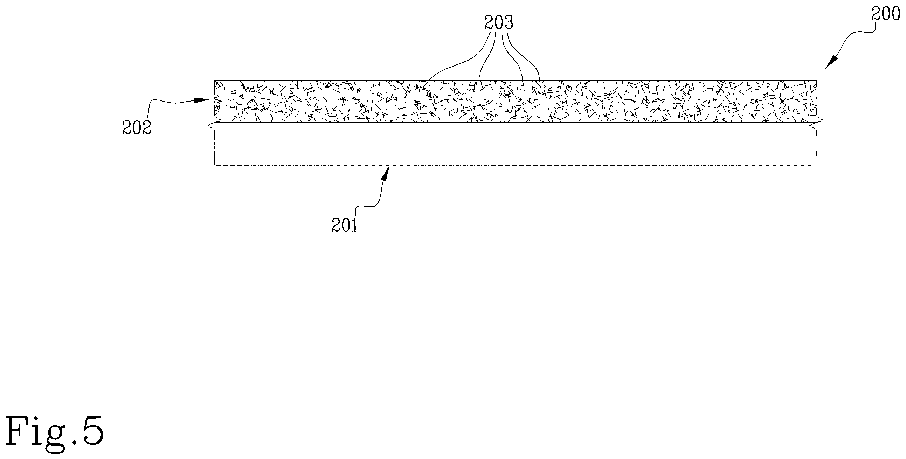

- figure 5 shows a diagrammatic side view of a segment separation device according to the preceding figures.

With reference to the accompanying figures, a segment separation device 100 from a continuous web 200 is indicated with reference numeral 1.

The continuous web 200 comprises, e.g., a layer 201 of non-woven fabric or woven fabric, a cellulose layer 202 and superabsorbent material 203 at least in part dispersed in the cellulose layer 202.

Similarly, the segments 100 obtained from the continuous web 200 comprise the layer 201 of non-woven fabric or fabric, the cellulose layer 202 and the superabsorbent material 203 at least in part dispersed in the cellulose layer 202. The segments 100 obtained from the continuous web 200 are, for example, absorbent pads intended for absorbent items such as, for example, underpads or diapers.

The separation device 1 comprises a flat advancement path P for the continuous web 200 and, at least in part, for the segments 100. The continuous web 200 and the segments 100 advance in an advancement orientation V.

The flat path P is preferably horizontal and constitutes the path along which the continuous web 200 advances and along which the segments 100 are cut from the continuous web.

The separation device 1 comprises a cutting knife 2, of a substantially known type, arranged along the advancement path P and configured to make weakening lines 101, 102, 103 on the continuous web 200.

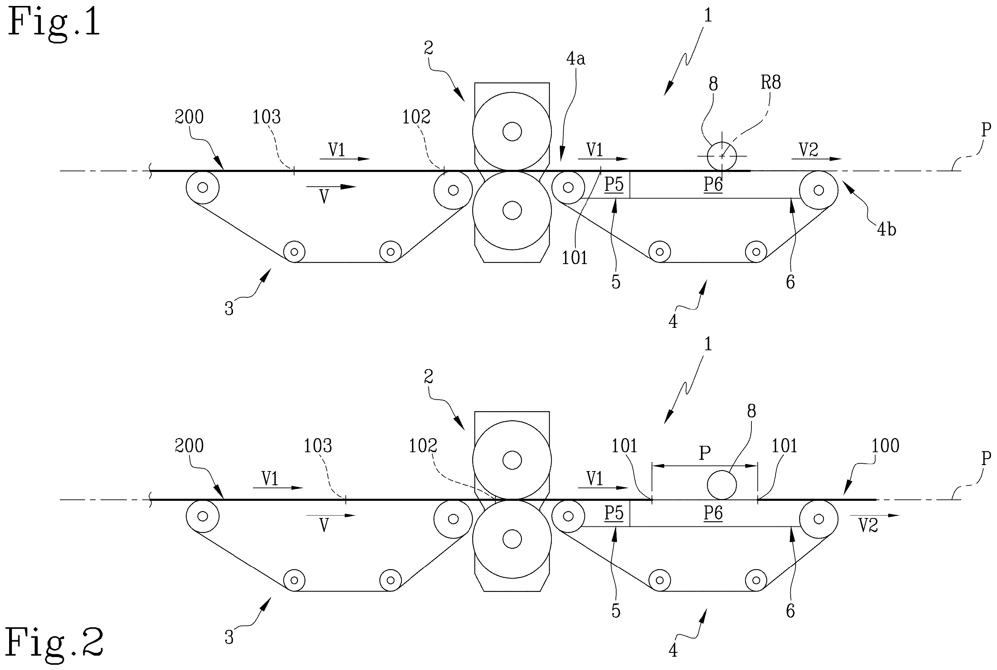

The weakening lines 102, 103 are indicated in dashed line in figures 1 and 2 because, although being shown, they are not yet made; in other words, reference numerals 102, 103 indicate the positions in which the cutting knife 2 will make the weakening lines 102, 103.

The cut made by the cutting knife 2 does not completely separate the sections 100 from one another, but makes a serrated or intermittent cut, at which the material is not completely separated but only scored, leaving connection points between one section 100 and the next one.

The separation device 1 comprises, upstream of the cutting knife 2 according to the advancement orientation V, a feeding system, indicated with reference 3 and diagrammatically shown as a substantially known movable aspirated belt, to feed, at a first speed V1, the continuous web 200 to the cutting knife 2.

The separation device 1 comprises, downstream of the cutting knife 2 according to the advancement orientation V, a first aspirated belt 4, arranged along the advancement path P.

The first aspirated belt 4 is movable at a second speed V2 greater than the first speed V1, and has an inlet 4a and an outlet 4b.

The first aspirated belt 4 is configured to receive the continuous web 200, provided with weakening lines 101, 102, 103, and advance it along the advancement path P.

The first aspirated belt 4 comprises a first aspirated part 5 and a second aspirated part 6, consecutive to the first aspirated part 5 according to the advancement orientation V.

The first aspirated part 5 has a first negative pressure of a first intensity P5 to withhold the continuous web 200 on the first aspirated belt 4.

The second aspirated part 6 has a second negative pressure of a second intensity P6 to withhold the continuous web 200 on the first aspirated belt 4.

The first intensity P5 is lower than the second intensity P6 so that the continuous web 200 is movable at the first speed V1 on the first aspirated part 5, sliding thereon, and is movable at the second speed V2 with the second aspirated part 6 towards the outlet of the first aspirated belt 4.

The continuous web 100 is accelerated on the second aspirated wall 6 and the acceleration completes the cutting of the continuous web at the weakening line 101 in the illustrated example.

In particular, the adhesion of the continuous web 200 on the second aspirated portion 6 increases as the continuous web 200 advances thereon, until it is brought to the second speed V2.

As shown, the segment 100, by virtue of the acceleration, is also spaced from the continuous web 200 by a pitch P corresponding preferably to the pitch of the products in which it will be incorporated.

With reference to figures 1 and 2, the first aspirated belt 4 is immediately downstream of the cutting knife 2, and the continuous web 200 is supported by the first aspirated belt 4 immediately after arranging the weakening lines 101, 102, 103.

In the illustrated embodiment, the separation device 1 comprises a roller 8 tangent to the first aspirated belt 4.

The roller 8 is rotatable about a rotation axis R8, orthogonal to the plane of figures 1 and 2, and configured to press the continuous web 200 on the first aspirated belt 4.

The roller 8 is preferably arranged at the second aspirated part 6 so as to cooperate with it in withholding the continuous web 200 during the acceleration that determines the tearing of the continuous web 200 at the weakening line 101, 102, 103.

Thereby, advantageously, there is a greater control at least of the position of the tear and, furthermore, by pressing the continuous web 200, it is possible to adopt a smaller negative pressure (with respect to a configuration without a roller), with a significant energy and cost saving.

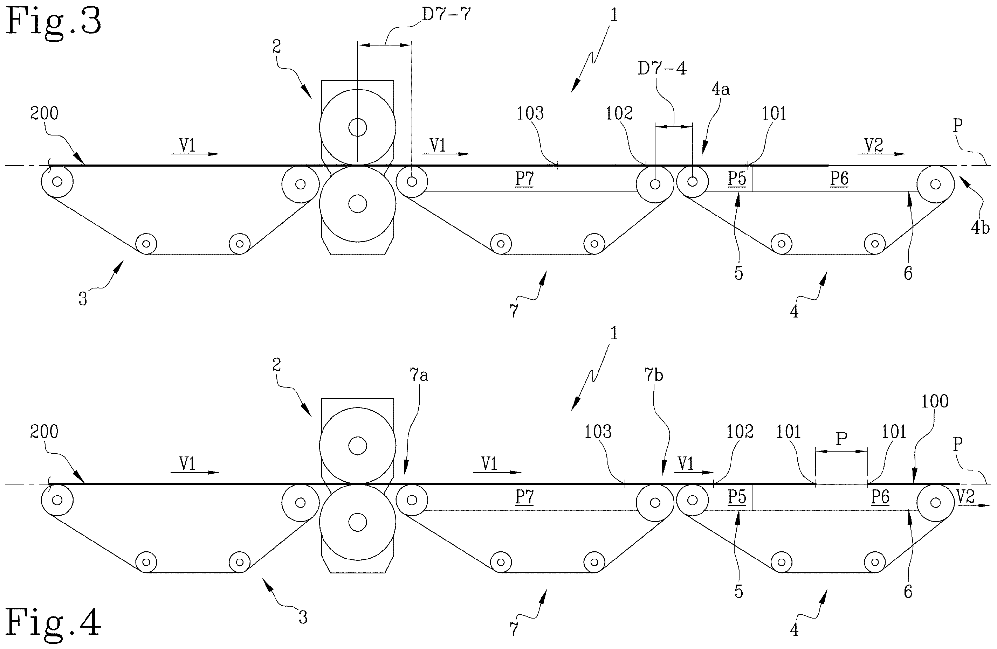

With reference to figures 3 and 4, the separation device 1 comprises a second aspirated belt 7, having a respective inlet 7a and a respective outlet 7b.

The second aspirated belt 7 is arranged along the advancement path P between the cutting knife 2 and the first aspirated belt 4.

The second aspirated belt 7 is movable at the first speed V1 and has a third negative pressure of a third intensity P7 to withhold the continuous web 200 and advance it along the advancement path P at the first speed V1.

In a preferred embodiment, a distance D2-7 along the advancement path P between the cutting knife 2 and the inlet 7a of the second aspirated belt 7 is greater than a distance D7-4 along the advancement path P between the outlet 7b of the second aspirated belt 7 and the inlet 4a of the first aspirated belt 4.

The separation of the segment 100 from the web 200 preferably occurs when the weakening line 101 that is intended to be torn is on the first aspirated portion 5 of the first aspirated belt 5 and the weakening line 102, consecutive to the weakening line 101 according to the advancement orientation V, is still on the second aspirated belt 7, as diagrammatically illustrated in the figure 3.

A segment separation method 100 from a continuous web 200 is hereinafter described. For the sake of convenience, the separation method is described with reference to device 1 without thereby losing generality.

The separation method comprises advancing the continuous web 200 in the advancement orientation V along the flat advancement path P, at a first speed V1.

The separation method comprises making at least one weakening line 101, 102, 103 on the continuous web 200.

The separation method comprises advancing the continuous web in the advancement orientation V along the flat advancement path P at the first speed V1, making on the continuous web 200 at least one first weakening line 101, 102, 103, and tearing the continuous web 200 at the first weakening line 101.

Tearing the continuous web 200 comprises accelerating the continuous web 200.

Preferably, the method comprises advancing the continuous web 200 at the first speed V1, preferably on a first flat movable surface, e.g. the first aspirated portion 5 of the first aspirated belt 4, in the advancement orientation V along the flat advancement path P and advancing the continuous web 200 on a second flat surface, e.g. the second aspirated portion 6 of the first aspirated belt 4, downstream of the first flat surface at a second speed V2 greater than the first speed V1 to tear the continuous web 200 at the weakening lines 101, 102, 103.

For the sake of simplicity, the first flat surface and the second flat surface are hereinafter also indicated with the same reference numerals 5, 6 as the first aspirated part and second aspirated part.

The first flat surface 5 and the second flat surface 6 are consecutive along the advancement path P and according to the advancement orientation V, so that the continuous web 200 is always supported and withheld.

Advancing the continuous web 200 on the second flat surface 6 occurs when the first weakening line 101 is on the first flat surface 5.

The separation method comprises sliding and withholding the continuous web 200, provided with the weakening lines 101, 102, 103, on the first movable flat surface 5 in the advancement orientation V along the flat advancement path, and accelerating the continuous web on the second flat surface 6 downstream of the first flat surface 5.

The continuous web 200 is advanced at speed V1 on the first flat surface 5, by sliding it thereon, which instead advances at speed V2. For example, the web 200 is withheld on the first flat surface by a negative pressure with intensity P5, smaller than intensity P6 of the negative pressure of the second flat surface 6.

As diagrammatically shown in figure 1, tearing the continuous web 200 at the first weakening line 101 occurs before making the second weakening line 102, consecutive to the first weakening line 101 according to the advancement orientation V.

The separation method provides that the segment 100 that precedes the weakening line 101 is mostly located on the second aspirated part 6 when the weakening line 101 is on the first aspirated part 5, and the weakening line 102 must still be formed, i.e., the section of the continuous web 200 in which the weakening line 102 will be cut is still upstream of the cutting knife 2.

In an embodiment illustrated in figures 3 and 4, the separation method comprises advancing the continuous web on a third flat surface, e.g., the second aspirated belt 7, to which explicit reference will be made hereinafter, being arranged upstream of the first flat surface 5 according to the advancement orientation V.

The separation method comprises advancing the continuous web on the third flat surface at the first speed V1 after making the sequence of weakening lines 101, 102, 103 on the continuous web 200 and before sliding the continuous web 200 provided with the weakening lines 101, 102, 103 on the first flat surface 5.

For example, the continuous web 200 is withheld on the third flat surface 7 so as to be movable with the same at the first speed V1.

As illustrated, accelerating the continuous web 200 on the second flat surface 5 occurs when the second weakening line 102, consecutive to the first weakening line 101 according to the advancement orientation V, is on the third flat surface 7, while the first weakening line which is torn is on the first flat surface 5.

A method for manufacturing an absorbent product comprising at least one permeable topsheet, an impermeable backsheet, and an absorbent pad inserted between the topsheet and the backsheet comprises a separation method for segments 100 from a continuous web 200, as described above.

The manufacturing method of the absorbing product comprises feeding the succession of absorbent pads 100, spaced therebetween, to an encounter with a second continuous web defining the backsheets and a third continuous web defining the topsheets, to obtain a continuous composite web formed of the second continuous web, the first continuous web and the absorbent pads withheld between the first and the second continuous web.

## Claims

1. A segment separation device (100) from a continuous web (200), said separation device comprising:
- a flat advancement path (P) of the continuous web (200);
- a cutting knife (2) arranged along the flat advancement path (P) and configured to make at least one weakening line (101, 102, 103) on the continuous web (200), said weakening line (101, 102, 103) being transverse to the continuous web (200) and located between consecutive segments (100) according to an advancement orientation (V) of the continuous web (200), said separation device comprising:
- a feeding system (3), upstream of the cutting knife (2) according to said advancement orientation (V) for feeding the continuous web (200) to the cutting knife (2) at a first speed (V1);
- a first aspirated belt (4) arranged downstream of the cutting knife (2) according to the advancement orientation (V) movable at a second speed (V2) greater than the first speed (V1), said first aspirated belt (4) having an inlet (4a) and an outlet (4b) for the continuous web (200) and being configured to receive the continuous web (200) provided with said weakening line (101, 102, 103) and advancing it along the flat advancement path (P) at said second speed (V2).

2. A separation device according to claim 1, wherein said first aspirated belt (4) comprises a first aspirated part (5) and a second aspirated part (6) downstream of the first aspirated part (5) according to the advancement orientation (V), said first aspirated part (5) having a first negative pressure of a first intensity (P5) for withholding the continuous web (200) on the first aspirated belt (4) and said second aspirated part (6) having a second negative pressure of a second intensity (P6) for withholding the continuous web (200) on the first aspirated belt (4), the first intensity (P5) being lower than the second intensity (P6) so that the continuous web (200) slides on the first aspirated part (5) and is movable with the second aspirated part (6) at said second speed (V2) towards the outlet of the first aspirated belt (4b).

3. A separation device according to claim 1 or 2, comprising a roller (8) tangent to the first aspirated belt (4) configured to press said continuous web onto said first aspirated belt (4).

4. A separation device according to claim 2, comprising a roller (8) tangent to the first aspirated belt (4) configured to press said continuous web (200) onto said first aspirated belt (4), said roller (8) being arranged at said second aspirated part (6) of the first aspirated belt (4).

5. A separation device according to any one of the preceding claims, wherein the inlet (4a) of the first aspirated belt (4) is immediately downstream and consecutive to the cutting knife (2) according to the advancement orientation (V).

6. A separation device according to any one of claims 1 to 4, comprising a second aspirated belt (7) arranged between the cutting knife (2) and the first aspirated belt (4), said second aspirated belt (7) having an inlet (7a) and an outlet (7b) and being movable at said first speed (V1) and generating a third negative pressure for withholding the continuous web (200) to advance it along the flat advancement path (P) at the first speed (V1).

7. A separation device according to claim 6, wherein a distance (D2-7) along the flat advancement path (P) between the cutting knife (2) and the inlet (7a) of the second aspirated belt (7) is greater than a distance (D7-4) along the flat advancement path (P) between the outlet (7b) of the second aspirated belt (7) and the inlet (4a) of the first aspirated belt (4).

8. A segment separation method (100) from a continuous web (200), said separation method comprising
advancing the continuous web (200) in an advancement orientation (V) along a flat advancement path (P) at a first speed (V1),
making, on the continuous web (200), at least a first weakening line (101, 102, 103) transverse to the continuous web (200) and located between consecutive segments (100) according to the advancement orientation (V), tearing the continuous web (200) at said first weakening line (101), tearing the continuous web (200) comprising accelerating the continuous web (200).

9. A separation method according to claim 8, comprising making the continuous web (200), provided with the first weakening line (101, 102, 103), slide on a first flat surface (5) movable in the advancement orientation (V) along the flat advancement path (P), accelerating the continuous web (200) comprising accelerating the continuous web (200) on a second flat surface (6) downstream of the first flat surface (5) to tear the continuous web (200) when said first weakening line (101) is located on said first flat surface (5).

10. A separation method according to claim 9, wherein making the continuous web (200), provided with the first weakening line (101, 102, 103), slide on the first flat surface (5) comprises withholding the continuous web (200) on the first flat surface (5) with a first negative pressure having a first intensity (P5) and accelerating the continuous web (200) on the second flat surface (6) comprises withholding the continuous web (200) on the second flat surface (6) with a second negative pressure having a second intensity (P6), said first intensity (P5) being lower than the second intensity (P6) so that the continuous web (200) slides on the first aspirated part (5) and is movable with the second aspirated part (6).

11. A separation method according to claim 9 or 10, comprising making a succession of weakening lines (101, 102, 103) on the continuous web (200),
advancing the continuous web (200) on a third flat surface (7) after making the succession of weakening lines (101, 102, 103) and before making the continuous web (200), provided with the weakening lines (101, 102, 103), slide on the first flat surface (5), the third flat surface (7) being upstream of the first flat surface (5) according to the advancement orientation (V), said succession of weakening lines comprising said first weakening line (101, 102, 103).

12. A separation method according to claim 11, wherein accelerating the continuous web (200) on the second flat surface (6) occurs when a consecutive second weakening line (102) of the first weakening line (101) according to the advancement orientation (V) is located on the third flat surface (7).

13. A separation method according to any one of claims 9 to 12, wherein tearing the continuous web (200) at the first weakening line (101) comprises advancing the continuous web (200), provided with the first weakening line (101, 102, 103), on the first flat surface (5) at a first speed (V1) and advancing the continuous web (200) on the second flat surface (6) at a second speed (V2) greater than the first speed (V1).

14. A separation method according to any one of claims 8 to 13, wherein tearing the continuous web at the first weakening line (101) occurs before making a second weakening line (102) consecutive of the first weakening line (101) according to the advancement orientation (V).

15. A separation method according to any one of claims 8 to 14, implemented with a device according to any one of claims 1 to 4.

16. A separation device according to any one of claims 1 to 7 and a separation method according to any one of claims 8 to 15, wherein said flat advancement path (P) is horizontal.

17. A manufacturing method for an absorbent product, comprising at least one permeable topsheet, an impermeable backsheet and an absorbent pad (100) inserted between the topsheet and the backsheet, said manufacturing method comprising a segment separation method from a continuous web (200) to make a succession of absorbent pads (100) spaced therebetween, feeding said succession of absorbent pads (100) spaced therebetween to an encounter with a second continuous web defining the backsheets and a third continuous web defining the topsheets, to obtain a continuous composite web formed of the second continuous web, the first continuous web and the absorbent pads (100) withheld between the first and second continuous web.
